# EUROPEAN PATENT APPLICATION

(11) **EP 1 410 759 A1**
(43) Date of publication of application: **21.04.2004**
(21) Application number: 02380213.5
(22) Date of filing: 17.10.2002
(51) Int. Cl.: A61B 5/08

(54) **Procedure for analysis of snoring and apnea and apparatus to carry out this analysis**

(71) Applicant: Sibel, S.A., 08026 Barcelona (ES); Hospital Universitari German Trias I Pujol, 08016 Badalona (ES); Universitat Politecnica de Catalunya, 08034 Barcelona (ES)
(72) Inventor: Campos, Raimon Jane, Dr., 08027 Barcelona (ES); Fiz Fernanez, José Antonio, Dr., 08850 Gava (ES); Morera I Prat, José, Dr., 08320 El Masnou (ES)
(74) Representative: Esteban Perez-Serrano, Maria Isabel

(57) **Abstract**

It is characterised in an apparatus which has a single sensor of respiratory sound, based on a microphone, with automatic detection of snoring episodes during sleep, temporal and individual analysis of these and diagnosis in real time of OSAS episodes, this apparatus incorporating a data acquisition unit, with memory card as well as, preferably, a PC, in addition to a power supply, signal conditioning means, an analogue-to-digital converter and a microcomputer.

## Description

### OBJECT OF THE INVENTION

The invention explained herein consists of a procedure for analysis of snoring and apnea and apparatus to carry out this analysis, of among the different methods and apparatuses intended for the ends mentioned.

This invention is characterised in a special construction of the apparatus which has a single sensor of respiratory sound, based on a microphone, with automatic detection of the snoring episodes during sleep, temporal and individual analysis thereof and diagnosis in real time of episodes of OSAS, this apparatus incorporating a data acquisition unit, with memory card as well as, preferably, a PC, besides a power supply, signal conditioning means, an analogue-to-digital converter and a microcomputer.

### BACKGROUND OF THE INVENTION

In the last 15 years, snoring has passed from being a socially tolerated occurrence to become an object of study and treatment. The possibility that snoring is one of the evolutionary manifestations of the Obstructive Sleep Apnea Syndrome (OSAS) indicates the interest in studying this sign by means of non-invasive techniques. It is calculated that the number of snorers in a normal population can lie between 50 and 80 percent thereof and of them around 10% have OSAS.

Of the general percentage of individual snorers, 20% seek help for heavy, habitual snoring of high sound intensity. It is to be pointed out that patients with OSAS have a higher morbidity and mortality, a greater risk of suffering traffic and workplace accidents, and episodes of cerebral and myocardial infarction which signifies a public health problem that ought to be addressed forthwith. However the public and private health services are overwhelmed by the present demand produced by said patients, which is creating long waiting lists and the impossibility of providing an adequate service. For this reason biomedical research is centring on the production of simple and easy-to-manage portable systems which at the same time offer great security in the diagnostic screening of these patients. This objective will be assisted by the important information that the acoustic signal of the snore can contribute, which objective is outlined in the present system.

The snore is a sound which is produced in the upper air tract during sleep which can appear when inhaling or exhaling, or in both events and the quality of which has a low tonality component. It can be preceded or not by an episode of apnea or hypopnea, as well as of guttural sounds or episodes of salivation. Snoring is generated fundamentally in the nasopharynx, in the oropharynx or in both places at the same time. Its intensity is always above that of the patient's normal breathing and in some patients can reach 80 dBA. Snoring can pass from being continuous or habitual to having an intermittent character accompanied by obstructive apneas at which point the habitual snorer has evolved toward the OSAS disorder. The causes of this change are not known, it being admitted that there exists a series of contributory factors such as obesity, arterial hypertension, the appearance of cerebral vascular incidents, and the consumption of drugs and alcohol. From the foregoing the need can be deduced for information with respect to the quantity and quality of the snores generated as well as the number of apneas occurring during the nocturnal study in order to be able to give a precise diagnosis of OSAS.

Up to now the systems of snore analysis on the market or in the elaboration process perform their function depending on complementary signals (nose-mouth flow, thorax - abdominal movements) which are necessary for the identification of "snoring" events.

This signifies that at least 3 sensors (thermistor, thorax-abdominal inductance bands, air flow into mouth) should be placed on the patient. These systems identify as a snore the event which is produced during the breathing cycle, measured by means of the sensors mentioned above.

When they have been identified, a subsequent analysis begins based mainly on the amplitude of the signal referred to a measured intensity and the frequency.

As distant background of the state of the art, mention is made of the US patent 4982738, of the firm Dr. Madaus GmbH, which incorporates an electret microphone in a band in laryngeal position and two detectors, accompanied by three electrocardiographic electrodes, two of them active and another for reference, with their corresponding detector of peaks in the signal to be evaluated, which involves an analysis procedure unavoidably different to that proposed.

Another patent, also a remote antecedent, is the WO 01/15602, an invention of Rembold Ch. and Suratt P., of a system and method for diagnosis of breathing disorders in patients by means of microphones and an analysis of the sounds emitted by these, so that high frequencies indicate the presence of these disorders.

The originality of the present procedure which is described below, rests on the principal fact that an intelligent system of identification is employed which dispenses with other complementary signals and which contributes sufficient data on the snore both in frequency and in time.

The employment of a single channel (microphone placed on the surface of the tracheal area) to identify and to analyse snoring and apnea represents a very significant advance in the analysis of this signal. It results in the handling of the system on the part of the doctor, technician or by the patient himself is straightforward, requiring for its operation no more than the positioning of the sensor at neck level and the instruction to begin acquisition.

### DESCRIPTION OF THE INVENTION

The present invention is characterised in a procedure for analysis of snoring and apnea and apparatus to carry out this analysis, of among all those different methods and apparatuses intended for these ends of identifying snoring.

The present procedure for the analysis and identification of snoring has as main contribution the technological innovation pursuant upon the development of modem techniques of analysis and identification of biological signals.

This system is devised to perform the following functions:
- Identification of the "snore" supplementary breathing noise, from other types of noise picked up by the microphone such as normal breathing, sounds of salivation, guttural sounds, surrounding noise, etc.
- Analysis of the snore in time, frequency and intensity and Identification thereof.
- Analysis and quantification of the number of apneas and hypopneas.

The system is oriented toward the following applications:
- Diagnostics and screening of patients suffering from OSAS.
- Control of the treatment of snoring patients and/or those suffering from OSAS that may undergo surgical intervention.
- Study of the evolution of snoring characteristics in patients that are habitual snorers and in patients suffering from OSAS.

Next some possible applications are explained which encompass a wide range of medical specialties and companies related with health although, by being a system that fundamentally identifies snoring qualitatively and quantitatively, the product will encounter its main user in all those professionally engaged in the diagnoses and treatment of patients with snoring problems:
- Doctors specialists in otorhinology: The snoring patient visits the otorhinologist seeking diagnosis and probable surgical treatment, by means of resection techniques, scar tissue formation and morphological changes. As is logical the effectiveness of the treatment has to be demonstrated by means of objective data which would in our case be given by our system. The positive changes from the surgical techniques are not evident until a minimum of three months has passed, therefore the carrying out of repeated polysomnogram studies signifies such a high cost that it appears unfeasible that follow-up of the patient by the specialist can be carried out with a minimum of guarantee. But without going further, the medical treatment of anatomic functional complaints of the upper air tract which cause snoring, such as a simple rhinitis or sinusitis, will require an objective control of the effectiveness of the treatment employed.
- Specialist pneumologists, internists: Specialist pneumologists and internists are consulted in their practice by patients suffering from heavy snoring and probable patients suffering from obstructive sleep apnea syndrome (OSAS). For this large group, besides quantifying snoring, it is important to perform the diagnosis of OSAS. The diagnosis can be obtained by approximation (by means of screening) or by objectifying the existence of nocturnal events of apnea - hypopnea. For this group both the diagnosis and the treatment are fundamental and need systems which objectify the nocturnal events. The conventional or the reduced polysomnogram are the usual procedures employed. All of them require more than one channel for quantifying and their positioning has to be carried out by a technician. Our system of one channel can provide a solution for the great demand existing to carry out these examinations as has already been explained above. The system is simple, easy to position and can be used in the patient's home. The OSAS disorder is treated by means of the application of mechanical systems (CPAP, BIPAP, etc.) or hygienic treatments. Said patients need to undergo a follow-up for the purpose of checking the effectiveness of the treatment applied, it being possible to use the present system.
- Endocrine specialists, dietitians: All that involved in weight loss therapy and the endocrinological dysfunctions associated with the OSAS disorder (hypothyroidism, etc.) are controlled by this group.
- Maxillary facial and digestive surgeons: The anatomical dysfunctions of the upper air tract are corrected by maxillary facial surgeons, and specialist otorhinologists. The gastric resection therapy applied to some morbid obesity cases in which the dietary treatment has been ineffective, is carried out by the digestive or general surgeons. The short and long term control of said therapies should be carried out by systems which quantify snoring and/or apnea.
- Family doctors and generalization of the system in primary attention. The family doctor or general practitioner is fully qualified to give an approximate diagnosis of the OSAS or to control the presence of snoring in his/her surgery and decide if the case should be seen by a specialist or is banal in nature.
- Other professionals: For pediatricians it is logical to think that it could be a useful means of diagnosing the risk of sudden infant death or OSAS without forgetting the functional problems of the upper air tract which produce heavy and evolutionary snoring. The medical internists will be able to use it as a monitor of pulmonary ventilation, the same as the sensors of flow, heat, movements, EMG activity are used on patients in intensive care units. Physiotherapists, that have to face the typical complaints of patients to whom they have to apply a muscle rehabilitation treatment and which is accompanied by OSAS or central apnea.
- Nursing graduates that carry out a job of applying simple techniques of diagnostics and treatment. The insurance inspectors when on legal grounds, objective reports are needed on the patients that may be candidates for a disability level or on the professionals that risk traffic or workplace accidents suffering from OSAS.
- Juridical appraisal: In the measure that heavy snoring is a cause of marriage annulment.

### DESCRIPTION OF THE DRAWINGS

The present descriptive specification is completed with a set of drawings which illustrate the preferred embodiment of the invention in a non-restrictive manner.

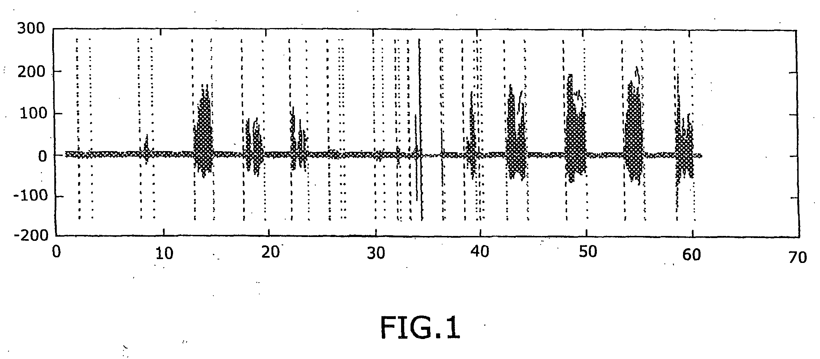

Figure 1 shows a diagram of the output of the detection block, with the waveform of the tracheal sound signal and the start and end marks of the snoring.

### PREFERRED EMBODIMENT OF THE INVENTION

In view of the foregoing, the present invention relates to a procedure for analysis of snoring and apnea and apparatus to carry out this analysis, of among the different methods and apparatuses which identify snoring.

Essentially the implementation of the following stages characterises this innovative method of analysis of the characteristics of snoring during sleep, in habitual snorers and in patients suffering from OSAS:
- The use of a single sensor (microphone placed on the surface of the tracheal area), for the acquisition of respiratory sound.
- The automatic detection of the snoring episodes during sleep.
- The temporal analysis (position, duration and intensity) in frequency and frequency-time of each snoring episode.

Additionally, this method is characterised in:
- The evaluation, of the effect of surgical techniques (specialties: otorhinology, maxillary facial, digestive) in the presence of snoring and the characteristics thereof, in accordance with the method described above.

This method is likewise characterised in the analysis of the presence of apnea and hypopnea, for the diagnosis of patients suffering from OSAS, in accordance with the following stages:
- Use of a single sensor (microphone placed on the surface of the tracheal area), for the acquisition of respiratory sound.
- Automatic detection of the snoring episodes during sleep.
- Temporal analysis (position, duration and type of episode), in frequency and frequency-time of each episode of apnea and hypopnea.

Finally it is characterised in the analysis of snoring, apnea or hypopnea, based on the respiratory sound signal, as described above, the carrying out thereof by means of a portable analysis unit in real time for screening patients suffering from OSAS.

To such ends the equipment mentioned consists of an acquisition unit, which picks up the signal from a microphone and stores it in a memory card so that, later or immediately, respectively, either an external computer or a PC incorporated in the equipment loads the data of the card in the memory, analyses them and records them, transmits them or, also optionally, prints them.

For this a power supply, signal conditioning means, an analogue-to-digital converter and a microcomputer are required.

As for the microphone, the electret type is recommended preferably, of diameter 5/6 mm, unidirectional type OBO 16BN - OC. This is located directly on the skin and is of encapsulated construction with an air chamber, in truncated conical form, 2 mm in height and of diameters 6 mm and 10 to 14 mm, depending on the case, the microphone response being optimised with these measurements with regard to sensitivity and frequency response. The encapsulation has an appropriate insulating material and is provided with a machined piece which incorporates four vents, this assembly being applied in paratracheal position, lateral to the cricoid cartilage, the same being held by means of a tape or an adhesive paper adapted to the dimensions of the microphone.

As for the signal conditioning, this starts with reception of the tracheal sound from the microphone in paratracheal position, lateral to the cricoid cartilage, secured with tape or adhesive paper of appropriate dimensions, the following measurement ranges being established: sound power level from 30 to 100 dB SPL and dynamic margin of 70 dB +/- 5 dB, as well as an S/N ratio better than 50 dB and a flat frequency response between 60 and 2000 Hz.

The software system is formed by an application and, also, by a set of libraries which serve for carrying out the analysis of the snoring, this last function being divided into two large blocks, that of detection and that of the analysis itself, the first incorporating a marking of the start and of the end of snoring and the second defining the parameters thereof.

The detection block is arranged in a segmentation stage and another for classification of a respiratory episode such as snoring, in accordance with a neural type network and with the following indications: respiratory sound, segmentation and detected snores, based on which the respiratory sound signal, loaded in a first data structure, is analysed to be broken down into diverse segments candidates for snoring and non-candidates for snoring, the segmenting element basing itself on the calculation of the signal envelope and the subsequent calculation of the entirety of those segments, in terms of the mean standard deviation of the background noise accompanying the signal.

For this, the algorithm carries out mainly an estimate of the variance of the signal in a window of a certain duration, and it is monitored over time. Starting from the information read, a threshold value is determined, which is adaptive in terms of the value of the present signal and of the recent history of the same signal making use of a forgetfulness factor.

This forgetfulness factor consists of giving more weight to the most recent episodes. This way, the threshold is adjusted to the variable conditions of the signal, however it is also protected against the presence of spurious values.

When the variance of the signal surpasses the threshold value, it is considered that the segment is a snoring episode candidate.

The segments considered as snoring candidates are likewise classified, incorporating also criteria of minimum duration of snoring, and criteria of grouping possible snoring episodes very near in time.

The neural type network, trained previously with diverse types of snoring, serves for defining the classification corresponding to the noise of the received segment, being loaded in a second data structure. Among the snoring candidate segments are the snores proper, breathing and apnea with noise.

The segments which are not strictly snores, as well as those others considered as not candidates for snoring (breathing; apnea without noise; not breathing without becoming apnea, according to a certain preset activity threshold), are analysed *a posteriori* as possible apnea episodes.

The algorithm employed contemplates criteria of temporal duration, environmental conditions, frequency characteristics, as well as the analysis itself carried out by the neural network.

In the current preferred embodiment, the algorithm employed is based on a feed-forward neural network. The input pattern of the network uses 22 temporal and spectral characteristics of the sound segment to be classified. The calculated pattern for each segment includes the following temporal characteristics: coefficients of the LPC parameters, zero crossings, power and tone; and, as frequency characteristics, the mean, variance, flatness, frequency of 50% power accumulation, frequency of 95% power accumulation, 100-500 Hz power ratio, power ratio in the range 100-500 Hz with respect to the range 55-2000 Hz, power ratio in the range 0-500 Hz with respect to the total power, power ratio in the range 0-500 Hz with respect to the power in the range 500-2000 Hz, standard deviation of the spectrum, bandwidth in which 95% of the energy is accumulated; absolute power in the bands I (0-150Hz), II (0-400Hz), III (400-2000Hz), IV (180-400Hz), as well as the power difference between bands II and III.

The information of the 22 parameters is used as input to the 22 neurons of the input layer. The neural system has 50 neurons in the hidden layer which process that information by means of the coefficients calculated with an adequate network training.

The output layer of the network consists of two neurons which allow classifying into a snoring or non-snoring episode (breathing, cough, voice...).

When the episode is classified as snoring, the entirety of the temporal parameters (start and end of the episode, mean and maximum intensity) are documented, as well as the frequency parameters which describe the snoring.

The snoring recording and analysis equipment is made up of a microphone, an analysis unit and another for acquisition. The microphone picks up the tracheal sound signal by means of a miniature microphone which is secured by means of tape or adhesive paper. The acquisition unit is formed by two subsystems, the first serves as support for the whole unit, to control it, manage the user interface and store the signal in a support. The analysis unit is formed by a hardware support wherein the software is run.

The acquisition unit consists of:
an analogue stage which consists of a bandpass filter and an amplifier which adapts the signal to the converter's margin
an analogue-to-digital converter, 12 bits, input range +5v to -5v
a RAM memory
EPROM memory and FLASH memory for storing the executable control program.
ATA Flash Memory
Control keyboard
Central processing unit, CPU
Power supply.

The analysis unit consists of a computer which can run the application capable of analysing, marking and classifying the events. The working procedure with the acquired signal is carried out as follows. The signal is in a unique file, wherefrom the main program will extract the signal and deliver it to an execution program which it has for 3 seconds to administer the necessary snoring. The program for classifying the events has a neural network of 3 layers.

The parameters that the equipment analyses are:

a)of each event.
Initial position of the Apnea
Duration of the Apnea
Type of the Apnea
Initial position of snoring
Duration of snoring
Type of snoring
Maximum intensity of snoring
Mean intensity of snoring
Peak frequency
Mean frequency
Centroid frequency
Maximum frequency
Standard deviation of the spectrum
Coefficient of symmetry of the spectrum
Flatness of the spectrum.

b) Parameters of the complete study.
Total number of snores.
Number of snores hourly
Total number of apneas
Mean intensity of snoring
Maximum intensity of snoring
Total time of snoring

c) Regarding the snoring pathology.
Graphic marker of the detected snores
Graphic marker of the detected apneas.
Graph of relationship of snoring/ study time
Cumulative graph of snoring/time.
Distribution table of snoring index.
Distribution table of mean power
Distribution table of percentage time snoring
Peak frequency
Mean frequency
Centroid frequency
Maximum frequency
Standard deviation
Symmetry coefficient
Flatness precision
Ratio of power above 800Hz
Ratio of power below 500 Hz
Power below 500 Hz
Fraction of power between 100 and 500 Hz
Ratio of power between 100 and 533 Hz. Etc.

d) Parameters of pulsoxymetry module.
Measurement range of SaO2 module
Range of tolerance of the heart pulse.

e) Parameters of position (possible values, supine, left side, right side and prone).

## Claims

1. Procedure for analysis of snoring and apnea, of among the different procedures which identify snoring based on sensors incorporated on the patient, essentially **characterised by** carrying out the following stages:
• The use of a single sensor (microphone placed in the surface of the tracheal area), for the acquisition of respiratory sound.
• The automatic detection of the snoring episodes during sleep.
• The temporal analysis (position, duration and intensity) frecuency and frequency-time of each snoring episode;
• carrying out both the appraisal of the effect of surgical techniques in the presence of snoring and the characteristics thereof as well as the analysis of the presence of apnea and hypopnea, for the diagnosis of patients suffering from OSAS, in accordance with the following stages:
• Use of a single sensor (microphone placed on the surface of the tracheal area), for the acquisition of respiratory sound.
• Automatic detection of the episodes of apnea and hipcapnea during sleep.
• Temporal analysis (position, duration and type of episode); of maximum and mean intensity of snoring; frecuency and frequency-time of each episode of apnea and hypopnea.

2. Procedure for analysis of snoring and apnea, according to the previous claim, **characterised in** establishing in the signal conditioning, as measurement ranges of sound power from 30 to 100 dB SPL and of dynamic margin of 70 dB +/- 5 dB, as well as a S/N ratio better than 50 dB and a flat frequency response between 60 and 2000 Hz.

3. Procedure for analysis of snoring and apnea, according to the first claim, **characterised in that** the snoring analysis is carried out in two large blocks, that of detection and that of the analysis proper, the first one incorporating a marking of the start and of the end of snoring and the second defining the parameters thereof.

4. Procedure for analysis of snoring and apnea, according to claims 1 and 3, **characterised in that** the detection block is arranged in a segmentation stage and another for classification of a breathing episode like snoring, in accordance with a neural type network and with the following indications: respiratory sound, segmentation and detected snores, based on which the respiratory sound signal is analysed in order to be broken down into diverse segments candidates for snoring and non-candidates for snoring, the segmenting element basing itself on the calculation of the signal envelope and the subsequent calculation of the entirety of those segments, in terms of the mean standard deviation of the background noise accompanying the signal.

5. Procedure for analysis of snoring and apnea, according to claims 1, 3 and 4, **characterised in that** the neural type network, trained previously with diverse types of snoring, serves for defining the classification corresponding to the noise of the received segment, being incorporated between the segments candidates for snoring, the snores proper, as well as breathing and apnea with noise.

6. Procedure for analysis of snoring and apnea, according to claims 1, 3 and 4, **characterised in that** the segments which are not strictly snores, as well as those others considered as non-candidates for snoring (breathing; apnea without noise; non-breathing without becoming apnea}, are analysed *a posteriori* as possible apnea episodes.

7. Procedure for analysis of snoring and apnea, according to the sixth claim, **characterised in that** the parameters of frequency analysis of each event are of peak, mean, centroid and maximum.

8. Procedure for analysis of snoring and apnea, according to the seventh claim, **characterised in that** the parameters of spectrum analysis are standard deviation, symmetry coefficient and flatness.

9. Procedure for analysis of snoring and apnea, according to the seventh claim, **characterised in that** the parameters of the power analysis are the power ratios above 800 Hz and below 500 Hz, the power with respect to the total is 500 Hz and the power fractions with respect to the total is between 100 Hz and 500 Hz, and with respect to the rest of the spectrum between 100 Hz and 500 Hz.

10. Procedure for analysis of snoring and apnea, according to the seventh claim, **characterised in that** the parameters of the complete study are the graphical markings differentiating post-apnea snoring with respect to the remainder; it likewise incorporates miscellaneous graphics, at least, that of snores / study time relationship and another accumulative of these relationships, miscellaneous tables, at least, that of distribution of the mean power by segments and that of distribution of the percentage of time snoring, in segments of 10 dB.

11. Procedure for analysis of snoring and apnea, according to claims 6, 9 and 10, **characterised in that** the frequency variables employed (peak, mean, centroid and maximum), as well as the standard deviation thereof, have accuracies of 1% and resolutions of 2.5 Hz.

12. Procedure for analysis of snoring and apnea, according to claims 6, 9 and 10, **characterised in that** the variables of symmetry coefficient; flatness; power ratios above 800 Hz and below 500 Hz; power below 500 Hz; fraction of power between 100 Hz and 500 Hz; as well as power ratio between 100 Hz and 500 Hz, have accuracies of +/- 5% and resolutions of 1%.

13. Procedure for analysis of snoring and apnea, according to claims 6, 9 and 10, **characterised in** the use as parameters of: the total number of snores; that of snores / hour; their mean and maximum intensities; the total time ratio of snoring / study time; as well as the snore pathology, with indication of the % of these which surpass a certain threshold of each parameter, in connection with the OSAS pathology.

14. Portable apparatus to carry out the analysis of snoring, apnea or hypopnea, based on the respiratory sound signal, of among the apparatuses which incorporate sensors on the patient, according to the method of the first claim, **characterised in** the carrying out of the analysis in real time of episodes of OSAS, based on:
• A microphone of paratracheal position, preferably of the electret type, of diameter 5/6 mm, unidirectional type OBO -16BN - OC, located directly on the skin and secured with a suitable tape or an adhesive paper, as well as encapsulated with an air chamber, in the shape of a truncated cone, 2 mm in height and of diameters 6 mm and 10 to 14 mm, with an appropriate insulating material, as well as provided with a piece which incorporates four venting holes.
• A data acquisition unit, which picks up the signal of a microphone and stores it in a memory card.
• A PC incorporated in the equipment which, in an immediate way, loads the data of the card in the memory, analyses them and records them, transmits them or, also, optionally, prints them.
• A power supply.
• Signal conditioning means.
• An analogue-to-digital converter.
• A microcomputer.

15. Portable apparatus to carry out the analysis of snoring, apnea or hypopnea, based on the respiratory sound signal, according to claim 2, **characterised in that**, alternatively to the PC incorporated in the portable equipment, it has an external computer which, subsequent to the data acquisition and the storage thereof in the memory card, processes the same and carries out real time analysis of OSAS episodes off-line.
